# EUROPÄISCHE PATENTSCHRIFT

(11) **EP 2 662 472 B1**
(45) Veröffentlichungstag und Bekanntmachung des Hinweises auf die Patenterteilung: **27.02.2019**
(21) Anmeldenummer: 13177354.1
(22) Anmeldetag: 22.03.2010
(51) Int. Cl.: C23C 18/12, A61M 15/00, A61M 11/00, A61M 11/06, B05B 11/00

(54) **Verfahren zur Beschichtung einer Oberfläche eines Bauteils**
Method for coating a surface of a component
Procédé de revêtement d'une surface d'un composant

(30) Priorität: 31.03.2009 EP 09156940
(43) Veröffentlichungstag der Anmeldung: 13.11.2013
(62) Teilanmeldung aus: 10709554.9
(73) Patentinhaber: BOEHRINGER INGELHEIM INTERNATIONAL GMBH, 55216 Ingelheim am Rhein (DE)
(72) Erfinder: Schuy, Steffen, 55216 Ingelheim am Rhein (DE); Meisenheimer, Martin, 55216 Ingelheim am Rhein (DE); Witte, Florian, 55216 Ingelheim am Rhein (DE)
(74) Vertreter: Freiherr von Foullon, Alexander

(56) Entgegenhaltungen:
- WO-A1-94/07607
- DE-A1- 10 300 983
- US-A1- 2007 264 437
- "Quantitative Online Detection of Low-Concentrated Drugs via a SERS Microfluidic System", CHEMPHYSCHEM, Bd. 8, Nr. 18, 5. Dezember 2007 (2007-12-05), Seiten 2665-2670, XP002605077,
- HENKEL T ET AL: "Chip modules for generation and manipulation of fluid segments for micro serial flow processes", CHEMICAL ENGINEERING JOURNAL, ELSEVIER SEQUOIA, LAUSANNE, CH LNKD- DOI:10.1016/J.CEJ.2004.01.021, Bd. 101, August 2004 (2004-08), Seiten 439-445, XP002594289, ISSN: 1385-8947 [gefunden am 2004-05-18]
- HAN Y ET AL: "Surface activation of thin silicon oxides by wet cleaning and silanization", THIN SOLID FILMS, ELSEVIER-SEQUOIA S.A. LAUSANNE, CH LNKD- DOI:10.1016/J.TSF.2005.11.048, Bd. 510, Nr. 1-2, 3. Juli 2006 (2006-07-03), Seiten 175-180, XP025006930, ISSN: 0040-6090 [gefunden am 2006-07-03]
- M. WANG, K. M. LIECHTI, Q. WANG, J. M. WHITE: "Self-Assembled Silane Monolayers: Fabrication with Nanoscale Uniformity", LANGMUIR, Bd. 21, Nr. 5, März 2005 (2005-03), Seiten 1848-1857, XP002605078,
- MANDAL SUPARNA ET AL: "Cytophobic surface modification of microfluidic arrays for in situ parallel peptide synthesis and cell adhesion assays", BIOTECHNOLOGY PROGRESS, AMERICAN INSTITUTE OF CHEMICAL ENGINEERS, US, Bd. 23, Nr. 4, Juli 2007 (2007-07), Seiten 972-978, XP009111799, ISSN: 8756-7938
- MYUNG-SUK CHUN ET AL: "Fabrication and validation of a multi-channel type microfluidic chip for electrokinetic streaming potential devices", LAB ON A CHIP: MINIATURISATION FOR CHEMISTRY, PHYSICS, BIOLOGY, MATERIALS SCIENCE AND BIOENGINEERING, Bd. 6, Nr. 2, Februar 2006 (2006-02), Seiten 302-309, XP055301968, GB ISSN: 1473-0197, DOI: 10.1039/b514327f

## Beschreibung

Die Erfindung bezieht sich auf ein Verfahren zur Beschichtung einer, insbesondere mikrostrukturierten, Oberfläche eines aus unterschiedlichen Werkstoffen, insbesondere Glas und Silizium, bestehenden Bauteils, bei dem zunächst die Oberfläche aktiviert und anschließend beschichtet wird, ein beschichtetes Bauteil und einen Zerstäuber mit einem beschichteten Bauteil.

In der WO 91/14468 A1 sowie der WO 97/12687 A1 ist jeweils ein Zerstäuber dargestellt, der unter dem Handelsnamen "Respimat" von der Boehringer Ingelheim Pharma GmbH & Co. KG in Form eines Inhalators angeboten wird. Der Zerstäuber dient zur treibgasfreien Verabreichung einer dosierten Menge eines flüssigen Arzneimittels zur inhalativen Anwendung. In dem Inhalator werden flüssige Arzneistoffformulierungen in einem Reservoir gelagert und über ein Steigrohr in eine Dosierkammer befördert, um schließlich durch eine Düse auszutreten.

Die Düse weist eine Flüssigkeitseinlassseite und eine Flüssigkeitsauslassseite auf. Auf der Flüssigkeitseinlassseite befindet sich eine Öffnung, durch die das von der Dosierkammer kommende flüssige Arzneimittel in die Düse eintreten kann. Auf der gegenüberliegenden Seite, der freien Stirnseite der Düse, tritt die Flüssigkeit dann durch zwei Düsenöffnungen aus, die so ausgerichtet sind, dass die aus den Öffnungen austretenden Flüssigkeitsstrahlen aufeinan- derprallen und dadurch zerstäubt werden. Die Düsenöffnungen sind in mindestens zwei aufeinanderliegenden Platten ausgebildet, von denen wenigstens eine eine Mikrostruktur aufweist, so dass die beiden fest miteinander verbundenen Platten auf einer Seite einen Flüssigkeitseinlass definieren, dem sich ein Kanal- und/oder Filtersystem anschließt, das in die Düsenöffnungen mündet. Die beiden Platten mit der Mikrostruktur und den Düsenöffnungen werden im Zusammenhang mit der Respimat-Technologie als Uniblock bezeichnet.

Ein derartiger Zerstäuber bringt zumeist Formulierungen auf Basis von Wasser oder Wasser-Ethanol-Gemischen aus und kann innerhalb kurzer Zeit eine kleine Menge der flüssigen Arzneistoffformulierung in der therapeutisch notwendigen Dosierung in ein therapeutisch-inhalativ geeignetes Aerosol vernebeln. Mit dem Zerstäuber können Mengen von weniger als 100 Mikroliter mit beispielsweise einem Hub zu einem Aerosol mit einer durchschnittlichen Teilchengröße von weniger als 20 Mikrometern so vernebelt werden, dass der inhalierbare Anteil des Aerosols bereits der therapeutisch wirksamen Menge entspricht.

Ein derartiger Zerstäuber bringt zumeist Formulierungen auf Basis von Wasser oder Wasser-Ethanol-Gemischen aus und kann innerhalb kurzer Zeit eine kleine Menge der flüssigen Arzneistoffformulierung in der therapeutisch notwendigen Dosierung in ein therapeutisch-inhalativ geeignetes Aerosol vernebeln. Mit dem Zerstäuber können Mengen von weniger als 100 Mikroliter mit beispielsweise einem Hub zu einem Aerosol mit einer durchschnittlichen Teilchengröße von weniger als 20 Mikrometern so vernebelt werden, dass der inhalierbare Anteil des Aerosols bereits der therapeutisch wirksamen Menge entspricht.

In dem Zerstäuber mit Respimat-Technologie wird die Arzneimittellösung mittels eines hohen Drucks von bis zu 300 bar in eine lungengängige Aerosolwolke niedriger Geschwindigkeit überführt, die dann vom Patienten eingeatmet werden kann. In seltenen Fällen können sich Rückstände aus der Formulierungslösung während der Benutzung des Zerstäubers als Verunreinigung an den Düsenauslässen anhaften, akkumulieren und zu einer Verlegung der Düsenauslässe führen, womit eine Ablenkung der Flüssigkeitsstrahlen und eine Veränderung des Feinpartikelanteils einhergehen kann. Auch wenn dieser Effekt bei einem Zerstäuber der Respimat-Technologie sehr selten ist, ist es aus Gründen der Qualitätssicherung ein Anliegen, Ablagerungen zu vermeiden.

Zur Vermeidung einer Verunreinigung einer Außenoberfläche des Düsensystems oder eines Mundstückes durch sich niedergeschlagene Flüssigkeit mit Feinpartikelanteilen schlägt die DE 103 00 983 A1 vor, dass die entsprechenden Flächen zumindest teilweise mikro- oder nanostrukturiert sind.

Die wissenschaftliche Veröffentlichung K. R. Ackermann, T. Henkel und J. Popp, "Quantitative Online Detection of Low-Concentrated Drugs via a SERS Microfluidic System", ChemPhysChem, 2007, 8, Seiten 2665 bis 2670 betrifft ein quantitatives Online-Monitoring von Konzentrationsfluktuationen verschiedener Wirkstoffe mittels oberflächenverstärkter Ramanstreuung in mikrofluidischen Bauteilen.

Weiterhin betrifft die wissenschaftliche Veröffentlichung T. Henkel, T. Bermig, M. Kielpinski, A. Grodrian, J. Metze und J.M. Köhler, "Chip modules for generation and manipulation of fluid segments for micro serial flow processes", Chemical Engineering Journal, 2004, 101, Seiten 439 bis 445 die sequenzierte Verarbeitung von kontinuierlichen Probenströmen in mikrofluidischer Umgebung, indem Proben durch Einführen einer Probenflüssigkeit in den kontinuierlichen Strom einer nicht mischbaren Trägerflüssigkeit erzeugt werden. Auf diese Weise werden sequenzierte Ströme in den Kapillaren des mikrofluidischen Bauteils erzeugt, welche für chemische Reaktionen oder analytische Zwecke genutzt werden können.

Weiterhin betrifft die WO 94/07607 A1 eine vereinfachte Form einer Düsenanordnung zur Herstellung von Sprays aus Flüssigkeiten, welche ein erstes Bauteil aufweist mit einer oder mehrerer Düsenauslassöffnungen, einer oder mehreren Flüssigkeitseinlässen und einem oder mehreren Kanälen, welche die Düsenauslassöffnung und den Flüssigkeitseinlass verbinden, wobei die Kanäle durch elektrisches oder chemisches Ätzen oder andere Verfahren, welche selektiv Material von der Oberfläche des Bauteils entfernen, hergestellt sind; ein zweites Bauteil, welches mit dem ersten Bauteil interagiert, um die Wände der Düsenauslassöffnung, des Flüssigkeitseinlasses und der Kanäle zu formen, so dass ein Flusspfad für Flüssigkeiten durch die Düsenanordnung entsteht.

Die wissenschaftliche Veröffentlichung Y. Han, D. Mayer, A. Offenhäusser und S. Ingebrandt, "Surface activation of thin silicon oxides by wet cleaning and silanization", Thin Solid Films, 2006, 510, Seiten 175 bis 180 betrifft die Oberflächenaktivierung dünner Siliziumoxidschichten durch Behandlung mit flüssigen Reinigungsmitteln und anschließende Silanisierung.

Die wissenschaftliche Veröffentlichung M. Wang, K.M. Liechti, Q. Wang und J.M. White, "Self-assembled silane monolayers: fabrication with nanoscale Uniformity", Langmuir, 2005, 21, Seiten 1848 bis 1857 betrifft die Herstellung gleichförmiger monomolekularer Lagen aus Silanen auf Siliziumoberflächen.

Darüber hinaus betrifft die wissenschaftliche Veröffentlichung S. Mandal, J.M. Rouillard, O. Srivannavit und E. Gulari, "Cytophobic surface modification of microfluidic arrays for in situparallel peptide synthesis and cell adhesion assays", Biotechnol. Prog. 2007, 23, Seiten 972 bis 978 die Einstellung von Oberflächen eines mikrofluidischen Chips im Hinblick auf die Bindung von Zellen mittels kombinierter Silan-PEG-Passivierung und anschließender Insitu-Festphasenpeptidsynthese.

Weiterhin betrifft die US 2007/0264437 A1 eine Beschichtungszusammensetzung aufweisend Silane mit mindestens einem hydrophoben Rest sowie mindestens einem hydrolysierbaren Rest.

Schließlich betrifft die wissenschaftliche Veröffentlichung M.-S. Chun, M.S. Shim und N.W. Choi, "Fabrication and validation of a multi-channel type microfluidic chip for electrokinetic streaming potential devices", Lab Chip, 2006, 6, Seiten 302 bis 309 einen hochdichten mikrofluidischen Mehrkanalchip, welcher in der Lage ist, durch das Helmholtz-Smoluchowski Prinzip ein elektrokinetisches Strömungspotential und eine äußere elektrische Spannung zu erzeugen.

Es ist Aufgabe der Erfindung, ein Verfahren und ein Bauteil sowie einen Zerstäuber mit einem Bauteil der eingangs genannten Art zu schaffen, das bzw. der selbstreinigend mit einer dünnen Funktionalisierungsschicht ausgebildet ist.

Erfindungsgemäß wird die Aufgabe durch ein Verfahren zur Beschichtung einer insbesondere mikrostrukturierten Oberfläche eines Bauteils gelöst, bei dem zunächst die Oberfläche aktiviert und anschließend beschichtet wird, wobei das Bauteil aus Glas und Silizium besteht und Oberflächen aus Glas und Silizium aufweist, wobei zur Aktivierung der Oberflächen aus Glas und Silizium eine oxidierende Lösung oder eine sauer-oxidierende Lösung verwendet wird, wobei zur Beschichtung der Oberflächen aus Glas und Silizium funktionelle Silane in unpolaren, aprotischen oder polaren, protischen Lösungsmitteln verwendet werden und wobei zur Beschichtung der Oberflächen aus Glas und Silizium das Bauteil in der Reaktionslösung mit Ultraschall behandelt wird.

Überraschenderweise wurde festgestellt, dass die zuvor erwähnten Lösungen zur Aktivierung von Oberflächen von zwei verschiedenen Materialien, nämlich Silizium und Glas, verwendet werden können, die zu einem monolithischen Bauteil gefügt sind. In der Literatur wird stets eine spezielle Lösung einem bestimmten Werkstoff zugeordnet, um die für eine Grenzflächenfunktionalisierung benötigten reaktiven Gruppen zu erzeugen, da die Aktivierung auf das jeweilige Substrat angepasst und spezifisch für unterschiedliche Materialien entwickelt werden muss. Beispielsweise erfolgt die Aktivierung der Oberfläche des Bauteils mit einer sogenannten Piranha-Lösung, nämlich der stark oxidierend wirkenden Peroxomonoschwefelsäure (konzentrierte Schwefelsäure: Wasserstoffperoxid-Lösung (30%) im Verhältnis 7:3), bei 70°C für 20 min. Alternativ kann die RCA-Reinigung angewandt werden, die üblicherweise bei der Wafer-Reinigung in der Mikroelektronik zum Einsatz kommt. Hierbei wird die folgende Lösung verwendet: Wasser:Ammoniak-Lösung (25%):Wasserstoffperoxid-Lösung (30%), Im Verhältnis 5:1:1. Das Bauteil wird der Lösung bei 75°C für 20 min. ausgesetzt.

Alternativ zur erfindundsgemäßen Lösung wird zur Aktivierung der Oberfläche eine basische Lösung in Form eines Küvettenreinigers verwendet. Vorzugsweise kommt der unter dem Handelsnamen "Hellmanex-Lösung" bekannte Küvettten- oder Glasreiniger der Firma Hellma GmbH & Co. KG, Müllheim, Deutschland, als wässrige 2%ige Lösung bei 70°C für 2x20 min im Ultraschallbecken zum Einsatz, wobei der Hersteller von dem Einsatz von Ultraschall im Zusammenhang mit seiner "Hellmanex-Lösung" abrät. Die "Hellmanex-Lösung" stellt eine Aktivierung sicher, die auf die unterschiedlichen Materialien, insbesondere Silizium und Glas, zur Erzeugung reaktiver Grenzflächen wirkt.

Zweckmäßigerweise wird die Oberfläche vor der Aktivierung gereinigt, insbesondere mittels Isopropanol und Wasser. Das Bauteil wird 5 min in Isopropanol und 5 min in Wasser getaucht. In Ausgestaltung wird die Oberfläche nach der Reinigung in einer Flusssäurelösung behandelt. Das Baden erfolgt für ca. 20 min in einer wässrigen 3% Flusssäurelösung (HF).

Erfindungsgemäß werden zur Beschichtung der Oberfläche funktionelle Silane in unpolaren, aprotischen oder polaren, protischen Lösungsmitteln verwendet. Die Beschichtung ist sehr dünn und erfolgt in einer Monolage. Das Bauteil mit seinen aktivierten Oberflächen wird bei einer toluolischen Reaktionsführung ca. 2h in eine 0,1 bis 1% Lösung von funktionellen Tricholosilane oder Dimethylmonochlorsilane in trockenen unpolaren, aprotischen Lösungsmitteln, wie Toluol, Benzol, Tetrachlorkohlenstoff, n-Alkane (C5-C10) oder dergleichen, eingetaucht. Anschließend wird das Bauteil in einem Stickstoffstrom getrocknet und ca. Ih bei etwa 120°C in einem Ofen getempert. überschüssige und nicht kovalent gebundene Rückstände werden nach dem Tempern mittels Toluol und Isopropanol von dem Bauteil gewaschen. Bei einer alkoholischen, insbesondere isopropanolischen, Reaktionsführung muss zunächst eine Reaktionslösung aktiviert werden, um eine Grenzflächenfunktionalisierung des Bauteils mit seinen aktivierten Oberflächen aus Silizium und Glas vornehmen zu können. Eine 0,1 bis 1% Lösung von funktionellen Triethoxysilanen in 2-Propanol/Wasser/Salzsäure (HCL) in einem Verhältnis 90 bis 98:2 bis 10:0,2 bis 0,5 wird ca. 5h bei Raumtemperatur gerührt. Anschließend wird das Bauteil mit seinen aktivierten Oberflächen für ca. 2h in die Lösung getaucht. Nach der Entnahme des Bauteils aus der Lösung wird die überschüssige Lösung schonend von dem Bauteil entfernt, was beispielsweise durch Abtropfen über einem saugfähigen Flies erfolgen kann. Danach wird das Bauteil bei etwa 120°C für ca. 2h getempert. überschüssige und nicht kovalent gebundene Rückstände werden nach dem Tempern mittels Isopropanol und Wasser von dem Bauteil gewaschen. Zur Beschichtung der Oberflächen werden die folgenden Stoffklassen eingesetzt:Perflouroalkylsilane, Alkylsilane, Aminoalkylsilane, Carbonsäurensilane, Trimethylammoniumsilane mit unterschiedlicher Kettenlänge, insbesondere C4-C18, wobei als funktionelle Silane reaktive Trihalogensilane (R1-Si-R₃, mit R=Cl, Br), Monohalogendimethylsilane (R1-Si(CH₃)₂R, mit R=Cl, Br) oder Trialkoxysilane (R1-Si-R₃, mit R= Methoxy- oder Ethoxy-Gruppen) verwendet werden.

Um eine gute Benetzung der zu beschichtenden Oberflächen des Bauteils sicherzustellen, wird erfindungsgemäß zur Beschichtung der Oberflächen das Bauteil in der Reaktionslösung mit Ultraschall behandelt. Durch die Ultraschallbehandlung wird insbesondere der Austausch der Reaktionslösung in den Kapillaren des Bauteils bewirkt.

Die Aufgabe wird bei dem Bauteil, das mindestens zwei fest miteinander verbundene Platten umfasst, von denen wenigstens eine Platte eine Mikrostruktur zur Ausbildung eines Kanal- und/oder Filtersystems und mindestens einer sich daran anschließenden Düsenöffnung aufweist, wobei die eine Platte aus Silizium und die andere Platte aus Glas besteht, dadurch gelöst, dass zumindest die Oberfläche der Mikrostruktur eine Beschichtung aufweist, die in dem zuvor erläuterten Verfahren aufgebracht ist. Durch diese Maßnahme ist eine Wechselwirkung von Bestandteilen und Partikeln der durch die Düsenöffnung ausgetragenen Formulierung mit den Grenzflächen des Bauteils unterbunden. Die freie Energie der Oberfläche und somit die Benetzbarkeit ist in diesem Bereich minimiert, womit eine Verminderung der Immobilisierung von Materialrückständen am Düsenauslass, also unmittelbar im Bereich der Düsenöffnung, einhergeht. Bei der Verwendung des Bauteils werden die Materialrückstände aus dem Bauteil ausgetragen und das beschichtete Bauteil ist durch den Einsatz der monomolekularen, kovalent gebundenen Antihaftschicht selbstreinigend. Die Funktionalisierung kann in Abhängigkeit von der Wechselwirkung unterschiedlicher Formulierungsbestandteile mit den Grenzflächen des Bauteils variabel geändert werden und beispielsweise hydrophile oder hydrophobe, positiv oder negativ geladene, oleophile oder oleophobe Eigenschaften aufweisen.

Erfindungsgemäß besteht die eine Platte aus Silizium und die andere Platte aus Glas. Die Platte aus Silizium ist vorzugsweise mit der Mikrostruktur versehen, weist also das Kanal- bzw. Filtersystem und die Düsenöffnungen auf und ist mit der Platte aus Glas verklebt.

Das Bauteil besteht aus den beiden fest miteinander verbundenen Platten aus Glas und Silizium, wobei die aus Silizium bestehende Platte einen oder mehrere mikrostrukturierte Kanäle aufweist, die die Einlassseite mit der Auslassseite verbinden. Auf der Auslassseite kann mindestens eine runde oder nicht-runde Düsenöffnung von 2 bis 10 µm Tiefe und 5 bis 15 µm Breite sein, wobei die Tiefe bevorzugt bei 4, 5 bis 6,5 µm und die Länge bei 7 bis 9 µm beträgt. Im Fall von mehreren Düsenöffnungen, bevorzugt sind zwei, können die Strahlrichtungen der Düsen im Bauteil, das im Wesentlichen einen Düsenkörper bildet, parallel zueinander verlaufen oder sie sind in Richtung Düsenöffnung gegeneinander geneigt. Bei einem Bauteil mit mindestens zwei Düsenöffnungen auf der Auslassseite können die Strahlrichtungen mit einem Winkel von 20° bis 160° gegeneinander geneigt sein, bevorzugt wird ein Winkel von 60° bis 150°, insbesondere bevorzugt 70° bis 100°. Die Düsenöffnungen sind bevorzugt in einer Entfernung von 10 bis 200 µm angeordnet, stärker bevorzugt in einer Entfernung von 10 bis 100 µm, besonders bevorzugt 30 bis 70 µm. Am stärksten bevorzugt sind 50 µm. Die Strahlrichtungen treffen sich dementsprechend in der Umgebung der Düsenöffnungen. Der Einfachheit halber wird im Folgenden eine Ausführungsform beschrieben, bei der lediglich die aus Silizium bestehende Platte des Bauteils reliefartige Mikrostrukturen aufweist, nicht jedoch die aus Glas bestehende Platte. In anderen Ausführungsformen ist die Situation gerade umgekehrt oder beide Platten weisen diese Mikrostrukturen auf. Auf der Silizium-Platte kann auf der ebenen Oberfläche ein Satz von Kanälen ausgebildet sein, um im Zusammenwirken mit der im Wesentlichen ebenen Oberfläche der Glas-Platte eine Vielzahl von Filterdurchgangswegen zu schaffen (Filterkanäle).

Daneben kann die Silizium-Platte eine Plenumkammer aufweisen, deren Decke wiederum durch die Glas-Platte gebildet ist. Die Plenumkammer kann den Filterkanälen vor- oder nachgeschaltet sein. Es können auch zwei derartige Plenumkammern ausgebildet sein. Ein anderer Satz von Kanälen auf der im Wesentlichen ebenen Oberfläche der Silizium-Platte, der den Filterkanälen nachgeschaltet ist, bildet zusammen mit der Glas-Platte einen Satz von Kanälen, die eine Vielzahl von Düsenauslassdurchgangswegen schaffen. Bevorzugt liegt der Gesamtquerschnittsflächenbereich der Düsenauslässe bei 25 bis 500µm². Der gesamte Querschnittsflächenbereich beträgt bevorzugt 30 bis 200 µm². In einer andere Ausführungsform weist auch diese Düsenkonstruktion nur eine einzige Düsenöffnung auf. In anderen Ausführungsformen dieser Art fehlen die Filterkanäle und/oder die Plenumkammer. Bevorzugt werden die Filterkanäle durch Vorsprünge gebildet, die zickzackförmig angeordnet sind. So bilden beispielsweise mindestens zwei Reihen der Vorsprünge eine solche Zick-Zack- Konfiguration. Auch können mehrere Reihen von Vorsprüngen ausgebildet sein, wobei die Vorsprünge jeweils seitlich zueinander versetzt sind, um dadurch zu diesen Reihen windschiefe weitere Reihen aufzubauen, wobei dann diese zuletztbeschriebenen Reihen die Zick-Zack-Konfiguration bilden. In solchen Ausführungsformen kann der Einlass und der Auslass jeweils einen Längsschlitz für unfiltriertes bzw. filtriertes Fluid aufweisen, wobei jeder der Schlitze im Wesentlichen genauso breit ist wie der Filter und im Wesentlichen genauso hoch ist wie die Vorsprünge auf den Einlass- bzw. Auslassseiten des Filters. Der Querschnitt der durch die Vorsprünge gebildeten Durchgangspassagen kann jeweils senkrecht zur Strömungsrichtung des Fluids stehen und kann - betrachtet in Strömungsrichtung - von Reihe zu Reihe abnehmen. Auch können die Vorsprünge, die näher zur Einlassseite des Filters angeordnet sind, größer sein als die Vorsprünge, die näher an der Auslassseite des Filters angeordnet sind. Daneben kann sich auch der Abstand zwischen der Silizium-Platte und der Glas-Platte in dem Bereich von der Einlassseite zur Auslassseite verjüngen. Die Zick-Zack-Konfiguration, die von den wenigstens zwei Reihen von Vor- sprüngen gebildet wird, weist einen Neigungswinkel von bevorzugt 20° bis 250° auf. Weitere Einzelheiten dieser Bauteilkonstruktion können der WO-94/07607 entnommen werden.

Schließlich wird die Aufgabe mit einem Zerstäuber zur Abgabe einer bestimmten Menge eines, insbesondere ein Arzneimittel aufweisenden, Fluids als Aerosol mit dem Bauteil der zuvor erläuterten Art gelöst.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigt:

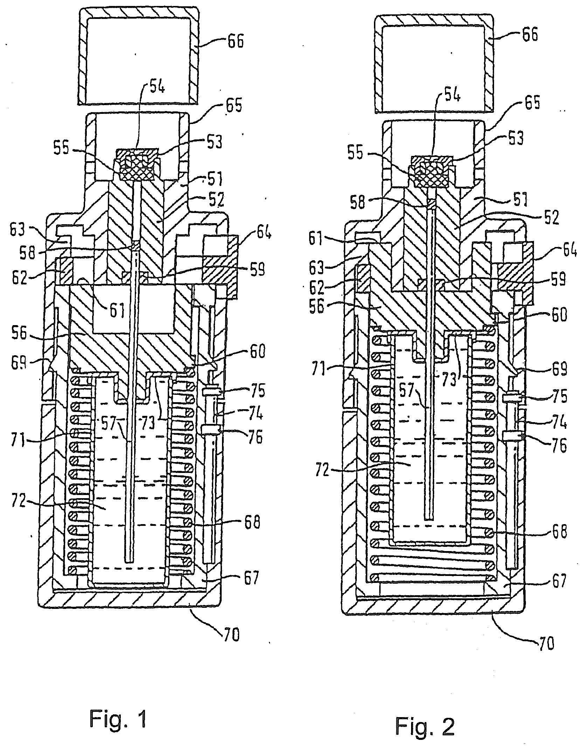

- Fig. 1: einen Längsschnitt durch einen erfindungsgemäßen Zerstäuber mit einer gespannten Feder,
- Fig. 2: einen Längsschnitt durch den Zerstäuber nach Fig. 1 mit entspannter Feder,

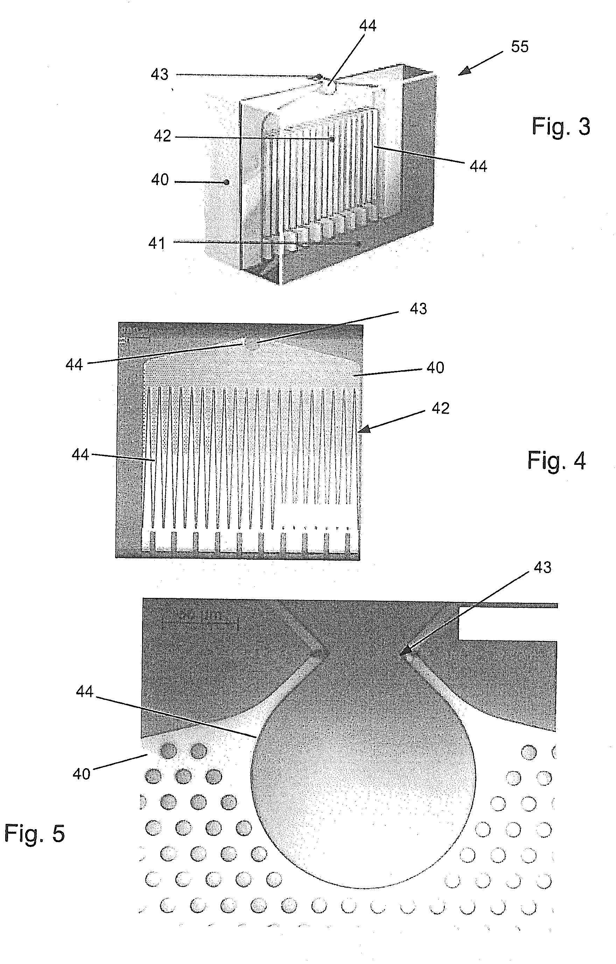

- Fig. 3: eine perspektivische Darstellung eines erfindungsgemäßen Bauteils,
- Fig. 4: eine Vorderansicht des Bauteils nach Fig. 3 und Fig. 5 eine vergrößerte Teildarstellung des Bauteils nach Fig. 3.
- Fig. 5: eine vergrößerte Teildarstellung des Bauteils nach Fig. 3.

Ein Gehäuseoberteil 51 des Zerstäubers umfasst ein Pumpen gehäuse 52, an dessen Ende ein Halter 53 für eine Zerstäuberdüse angebracht ist. In dem Halter 53 befindet sich eine sich erweiternde Ausnehmung 54 und das als Düsenkörper ausgebildete Bauteil 55. Ein in einem Abtriebsflansch 56 eines Sperrspannwerkes befestigter Hohlkolben 57 ragt teilweise in einen Zylinder des Pumpengehäuses 52 hinein. An seinem Ende trägt der Hohlkolben 57 einen Ventilkörper 58. Der Hohlkolben 57 ist mittels einer Dichtung 59 abgedichtet. Innerhalb des Gehäuseoberteils 51 befindet sich ein Anschlag 60 des Abtriebsflanschs 56, an dem eine Druckfeder 68 anliegt. Nach dem Spannen der Druckfeder 68 schiebt sich ein Sperrglied 62 zwischen einen Anschlag 61 des Abtriebsflanschs 56 und eine Abstützung 63 im Gehäuseoberteil 51. Eine Auslösetaste 64 steht mit dem Sperrglied 62 in Verbindung. Das Gehäuseoberteil 51 endet in einem Mundstück 65 und ist mit einer aufsteckbaren Schutzkappe 66 verschlossen.

Ein Federgehäuse 67 mit der Druckfeder 68 ist mittels Schnappnasen 69 und einem Drehlager an dem Gehäuseoberteil 51 drehbar gelagert. Über das Federgehäuse 67 ist ein Gehäuseunterteil 70 geschoben und innerhalb des Federgehäuses 67 befindet sich ein Vorratsbehälter 71 für zu zerstäuben- des Fluid 72. Der Vorratsbehälter 71 ist mit einem Stopfen 73 verschlossen, durch den der Hohlkolben 57 in den Vorratsbehälter 71 hineinragt und mit seinem Ende in das Fluid 72, also den Vorrat an Wirkstofflösung, eintaucht.

In einer Mantelfläche des Federgehäuses 67 ist eine Spindel 74 für ein mechanisches Zählwerk angebracht (optional). An dem dem Gehäuseoberteil 51 zugewandten Ende der Spindel 74 befindet sich ein Antriebsritzel 75. Auf der Spindel 74 sitzt ein Reiter 76. Das Bauteil 55 - ein so genannter Uniblock - umfasst zwei fest miteinander verbundene Platten 40, 41, von denen die eine Platte 40 aus Silizium fertigt ist und eine Mikrostruktur 42 zur Ausbildung eines Kanal- bzw. Filtersystems und einer sich daran anschließenden Düsenöffnung 43 auf- weist. Die Platte 40 aus Silizium ist auf der Seite mit der Mikrostruktur 42 mit der aus Glas gefertigten Platte 41 fest verbunden.

Zumindest die Oberfläche 44 der Mikrostruktur 42 weist eine aus funktionellen Silanen bestehende Beschichtung auf, die eine Wechselwirkung von Bestandteilen und Partikeln der durch die Düsenöffnung 43 ausgetragenen Formulierung mit den Grenzflächen des Bauteils 55 unterbindet. Bei der Verwendung des Zerstäubers mit dem Bauteil 55 werden die Materialrückstände aus dem Bauteil 55 ausgetragen und das beschichtete Bauteil 55 ist durch den Einsatz der monomolekularen, kovalent gebundenen Antihaftschicht selbstreinigend.

Die Oberflächen 44 des Bauteils 55, insbesondere der Mikrostruktur 42, werden zunächst gereinigt, in dem das Bauteil 5 zuerst 5 min in Isopropanol und anschließend 5 min in Wasser getaucht wird. Danach wird das Bauteil 55 für ca. 20 min in einer wässrigen 3% Flusssäurelösung (HF) gebadet.

Die eigentliche Aktivierung der Oberfläche 44 erfolgt in nicht erfindungsgemäßer Weise in Form eines vergleichbaren Referenzbeispiels mit einem Küvettenreiniger, wobei insbesondere der unter dem Handelsnamen "Hellmanex-Lösung" bekannte Küvettten- oder Glasreiniger der Firma Hellma GmbH & Co. KG, Müllheim, Deutschland, als wässrige 2%ige Lösung bei 70°C für 2x20 min im Ultraschallbecken zum Einsatz kommt.

Zur Funktionalisierung wird beispielsweise 1H,1H,2H,2H- Perfluorooctyltriethoxysilan verwendet, das beispielsweise unter dem Handelsnamen Dynasylan von der Fa. Evonik AG, Düsseldorf, vertrieben wird. Nachdem das Bauteil 55 unter Einwirkung von Ultraschall ca. 2h einer Lösung mit dem funktionellen Silan ausgesetzt ist, lässt man die überschüssige Lösung von dem Bauteil 55 abtropfen und das Bauteil wird ca. 1 bis 2h bei 120°C in einem Ofen getempert. überschüssige und nicht kovalent gebundene Rückstände werden nach dem Tempern mittels Isopropanol und Wasser von dem Bauteil 55 gewaschen.

## Patentansprüche

1. Verfahren zur Beschichtung einer, insbesondere mikrostrukturierten, Oberfläche (44) eines Bauteils (55), bei dem zunächst die Oberfläche (44) aktiviert und anschließend beschichtet wird,
**dadurch gekennzeichnet,**
**dass** das Bauteil (55) aus Glas und Silizium besteht und Oberflächen aus Glas und Silizium aufweist,
**dass** zur Aktivierung der Oberflächen (44) aus Glas und Silizium eine oxidierende Lösung oder eine sauer-oxidierende Lösung verwendet wird,
**dass** zur Beschichtung der Oberflächen (44) aus Glas und Silizium funktionelle Silane in unpolaren, aprotischen oder polaren, protischen Lösungsmitteln verwendet werden und
**dass** zur Beschichtung der Oberflächen (44) aus Glas und Silizium das Bauteil (55) in der Reaktionslösung mit Ultraschall behandelt wird.

2. Verfahren nach Anspruch 1, **dadurch gekennzeichnet, dass** zur Aktivierung der Oberfläche (44) als oxidierende Lösung eine Lösung bestehend aus Wasser, Ammoniak-Lösung und Wasserstoffperoxid-Lösung vorzugsweise im Verhältnis 5:1:1, besonders bevorzugt gemäß des RCA-Reinigungs-Verfahrens verwendet wird.

3. Verfahren nach Anspruch 1, **dadurch gekennzeichnet, dass** zur Aktivierung der Oberfläche (44) als sauer-oxidierende Lösung eine Piranha-Lösung verwendet wird.

4. Verfahren nach einem der vorangegangenen Ansprüche, **dadurch gekennzeichnet, dass** die Oberfläche (44) vor der Aktivierung gereinigt wird, insbesondere mittels Isopropanol und Wasser.

5. Verfahren nach Anspruch 4, **dadurch gekennzeichnet, dass** zur Beschichtung der Oberfläche (44) Alkysilane verwendet werden.

6. Verfahren nach Anspruch 4 oder 5 **dadurch gekennzeichnet, dass** zur Beschichtung der Oberfläche Perfluoroalkylsilane, Trihalogensilane oder Monohalogendimethylsilane eingesetzt werden.

7. Verfahren nach einem der Ansprüche 4 bis 6 **dadurch gekennzeichnet, dass** Dimethylmonochlorsilane in trockenen unpolaren, aprotischen Lösungsmitteln zur Beschichtung verwendet werden.

8. Verfahren nach Anspruch 7 **dadurch gekennzeichnet, dass** zur Beschichtung das Bauteil (55) in das Lösungsmittel mit dem Dimethylmonochlorsilan eingetaucht wird und anschließend im Stickstoffstrom getrocknet wird

9. Verfahren nach Anspruch 5 **dadurch gekennzeichnet, dass** eine Lösung von Triethoxysilanen verwendet wird.

10. Verfahren nach Anspruch 9 **dadurch gekennzeichnet, dass** eine 0,1 bis 1% Lösung von Triethoxysilanen in 2 Propanol/Wasser/Salzsäure vorzugsweise ca. 5 Stunden gerührt wird und anschließend zur Beschichtung das Bauteil mit seinen aktivierten Oberflächen in die Lösung eingetaucht wird.

11. Verfahren nach Anspruch 9 oder 10 **dadurch gekennzeichnet, dass** nach der Entnahme des Bauteils aus der Lösung überschüssige Lösung durch Abtropfen entfernt wird.

12. Verfahren nach einem der vorangehenden Ansprüche, **dadurch gekennzeichnet, dass** das Bauteil (55) nach der Beschichtung getempert wird.

13. Bauteil, das mindestens zwei fest miteinander verbundene Platten (40, 41) umfasst, von denen wenigstens eine Platte (40) eine Mikrostruktur (42) zur Ausbildung eines Kanal- und/oder Filtersystems und mindestens einer sich daran anschließenden Düsenöffnung (43) aufweist, wobei die eine Platte (40) aus Silizium und die andere Platte (41) aus Glas besteht, wobei zumindest die Oberfläche (44) der Mikrostruktur (42) eine Beschichtung aufweist, die in einem Verfahren nach einem der Ansprüche 1 bis 12 aufgebracht ist.

14. Zerstäuber zur Abgabe einer bestimmten Menge eines, insbesondere ein Arzneimittel aufweisenden, Fluids (72) als Aerosol mit einem Bauteil (55) nach Anspruch 13.

## Claims

1. A method for coating a surface (44), particularly a microstructured surface, of a component (55), where the surface (44) is firstly activated and then coated,
**characterised in**
**that** the component (55) consists of glass and silicon and has surfaces made of glass and silicon,
**that** for activation of the surfaces (44) of glass and silicon, an oxidising solution or an acid-oxidising solution is used,
**that** for coating the surfaces (44) of glass and silicon, functional silanes are used in non-polar, aprotic or polar, protic solvents, and
**that** for coating the surfaces (44) of glass and silicon, the component (55) is treated in the reaction solution by means of ultrasound.

2. The method according to claim 1, **characterised in that** for the activation of the surface (44), a solution consisting of water, ammonia solution and hydrogen peroxide solution, preferably in a ratio of 5:1:1, particularly preferably according to the RCA Clean method, is used as the oxidising solution.

3. The method according to claim 1, **characterised in that** for the activation of the surface (44), a piranha solution is used as the acid-oxidising solution.

4. The method according to one of the preceding claims, **characterised in that** the surface (44) is cleaned before activation, particularly by means of isopropanol and water.

5. The method according to claim 4, **characterised in that** alky silanes are used for coating the surface (44).

6. The method according to claim 4 or 5, **characterised in that** perfluoroalkyl silanes, trihalogen silanes or monohalogen dimethylsilanes are used for coating the surface.

7. The method according to one of claims 4 to 6, **characterised in that** dimethyl-monochlorsilanes in dry, non-polar, aprotic solvents are used for coating.

8. The method according to claim 7, **characterised in that**, for coating, the component (55) is immersed in the solvent with the dimethylmonochlorsilane and then dried in the nitrogen stream.

9. The method according to claim 5, **characterised in that** a solution of triethoxysilanes is used.

10. The method according to claim 9, **characterised in that** a 0.1 to 1% solution of triethoxysilanes is stirred preferably for approx. 5 hours into 2 propanol/water/hydrochloric acid and then the component is immersed into the solution with its activated surfaces for coating.

11. The method according to claim 9 or 10, **characterised in that** excess solution is removed by means of dripping after the component is removed from the solution.

12. The method according to one of the preceding claims, **characterised in that** the component (55) is tempered after the coating.

13. A component comprising at least two fixedly interconnected panels (40, 41), of which at least one panel (40) has a microstructure (42) for forming a duct and/or filter system and at least one adjoining nozzle inlet (43), wherein the one panel (40) consists of silicon and the other panel (41) of glass, wherein at least the surface (44) of the microstructure (42) has a coating, which is applied in a method according to one of claims 1 to 12.

14. An atomiser for the delivery of a specific quantity of a fluid (72), particularly containing a medicinal product, as an aerosol, with a component (55) according to claim 13.

## Revendications

1. Procédé de revêtement d'une surface (44), en particulier micro-structurée, d'un composant (55) dont la surface (44) est d'abord activée et ensuite revêtue,
**caractérisé**
**en ce que** le composant (55) est constitué de verre et de silicium et les surfaces présentent du verre et du silicium,
**en ce que** pour l'activation de la surface (44) en verre et en silicium on utilise une solution oxydante ou une solution oxydante-acide,
**en ce que** pour le revêtement de la surface (44) en verre et en silicium on utilise des silanes fonctionnels dans des solvants non polaires, aprotiques ou polaires, protiques, et
**en ce que** pour le revêtement de la surface (44) en verre et en silicium le composant (55) est traité par ultrasons dans la solution de réaction.

2. Procédé selon la revendication 1, **caractérisé en ce que** pour l'activation de la surface (44) on utilise comme solution oxydante une solution constituée d'eau, de solution d'ammoniac et de solution de péroxyde d'hydrogène de préférence dont le rapport est 5:1:1, de préférence encore selon le procédé de nettoyage RCA.

3. Procédé selon la revendication 1, **caractérisé en ce que** pour l'activation de la surface (44) on utilise une solution Piranha comme solution oxydante-acide.

4. Procédé selon l'une des revendications précédentes, **caractérisé en ce que** la surface (44) est nettoyée avant l'activation, en particulier à l'aide d'isopropanol et d'eau.

5. Procédé selon la revendication 4, **caractérisé en ce qu'**on utilise des alkylsilanes pour le revêtement de la surface (44).

6. Procédé selon la revendication 4 ou 5, **caractérisé en ce qu'**on utilise des perfluoroalkylsilanes, des trihalogénosilanes ou des monohalogénodiméthylsilanes pour le revêtement de la surface.

7. Procédé selon l'une des revendications 4 à 6, **caractérisé en ce que** les diméthyle monochlorosilanes sont utilisés dans des solvants secs aprotiques, non polaires pour le revêtement.

8. Procédé selon la revendication 7, **caractérisé en ce que** pour le revêtement le composant (55) est immergé dans le solvant avec le diméthylmonochlorosilane et ensuite est séché dans le flux d'azote. Procédé selon la revendication 5 **caractérisé en ce qu'**une solution de triéthoxysilanes est utilisée.

9. Procédé selon la revendication 5, **caractérisé en ce qu'**une solution de triéthoxysilanes est utilisée.

10. Procédé selon la revendication 9, **caractérisé en ce qu'**une solution entre 0,1 et 1 % de triéthoxysilanes est mélangée dans 2 acide chlorhydrique/eau/propanol de préférence pendant 5 heures et ensuite le composant est immergé pour le revêtement avec ses surfaces activées dans la solution.

11. Procédé selon la revendication 9 ou 10, **caractérisé en ce que**, après le retrait du composant de la solution, la solution excédentaire est éliminée par égouttage.

12. Procédé selon l'une des revendications précédentes, **caractérisé en ce que** le composant (55) est recuit après le revêtement.

13. Composant, qui comprend au moins deux plaques (40, 41) reliées à demeure l'une à l'autre, parmi lesquelles au moins une plaque (40) présente une micro-structure (42) pour former un système de filtre et/ou de canal et au moins un orifice de buse (43) s'y raccordant, ladite plaque (40) étant constituée de silicium et l'autre plaque (41) de verre, au moins la surface (44) de la micro-structure (42) présentant un revêtement qui est appliqué selon un procédé selon l'une des revendications 1 à 12.

14. Brumisateur de distribution d'une quantité déterminée d'un fluide (72) comportant en particulier un médicament comme aérosol, comprenant un composant (55) selon la revendication 13.
